(19)

Europäisches Patentamt
European Patent Office
Office européen des brevets

(11) **EP 2 100 522 A1**

(12) **EUROPEAN PATENT APPLICATION**

(43) Date of publication:
**16.09.2009 Bulletin 2009/38**

(51) Int Cl.:
*A23L 1/304* (2006.01)          *A61K 33/04* (2006.01)
*C01B 19/00* (2006.01)          *C01B 19/02* (2006.01)
*C01F 11/18* (2006.01)

(21) Application number: **08290234.7**

(22) Date of filing: **12.03.2008**

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(71) Applicants:
• **UNIVERSITE JOSEPH FOURIER**
  **F-34000 Saint-Martin D'Heres (FR)**
• **Centre National de la Recherche Scientifique (CNRS)**
  **75794 Paris Cedex 16 (FR)**

(72) Inventors:
• **Montes Hernandez, German**
  **38470 Vinay (FR)**
• **Charlet, Laurent**
  **38660 Le Touret (FR)**
• **Renard, François**
  **38400 Saint-Martin d'Hères (FR)**

(74) Representative: **Grosset-Fournier, Chantal Catherine et al**
  **Grosset-Fournier & Demachy**
  **54, Rue Saint-Lazare**
  **75009 Paris (FR)**

(54) **New selenium containing composites, their use and a process for preparing the same**

(57)     The invention relates to the use of a seleno-L-cystine or a derivative thereof in an aqueous suspension of $Ca(OH)_2$ or $Mg(OH)_2$, for preparing an initial dispersion of seleno-L-cystine in said aqueous suspension, said seleno-L-cystine being liable to be fragmented under an appropriate concentration of $O_2$ and to be used for the preparation of a selenium containing composite or compositions containing said composite.

EP 2 100 522 A1

**Description**

The invention relates to new selenium containing composites, their use and a process for preparing the same.

Selenium is well known for its photochemical and semiconductor properties and has been successfully used in solar cells, rectifiers, photographic exposure meters and xerography ( J. M. Song, J. H. Zhu, S. H. Yu, J. Phys. Chem. B. 110 (2006) 23790; Y. Ding, Q. Li, Y. Jia, L. Chen, J. Xing, Y. Qian, J. Crystal Growth 241 (2002) 489; Y. Bai, Y. Wang, Y. Zhou, W. Li, W. Zheng, Materials Letters (2007) doi:10.1416/j.matlet.2007.11.098). Selenium is also a key trace element required in small quantities in humans and animals for the function of a number of selenium-dependent enzymes, such as glutathione peroxidase (GPX) and thioredoxin reductase; however this element can also be toxic in larger doses.

Both the beneficial and toxic effects of selenium are based on concentration ingested and on its chemical forms (E. Kapolna, M. Shah, J. A. Caruso, P. Fodor, Food Chemistry 101 (2007) 1398; A. Tarze, M. Dauplais, I. Grigoras, M. Lazard, N. T. Ha-Duong, F. Barbier, S. Blanquet, P. Plateau, J. Biol. Chem. 282/12 (2007) 8759; R. Caputo, S. Capone, M. Della Greca, L. Longobardo, G. Pinto, Tetrahedron Letters 48 (2007) 1425; D. Peng, J. Zhang, Q. Liu, E. Will Taylor, J. Inorg. Biochem. 101 (2007) 1457; J. E. Spallholz, D. J. Hoffman, Aquatic Toxicology 57 (2002) 27; M. S. Stewart, J. E. Spallholz, K. H. Neldner, B. C. Pence, Free Radic. Biol. Med 26 (1999) 42; M. Ochsenkuhn-Petropoulou, F. Tsopelas, Anal. Chim. Acta 467 (2002) 167).

Inorganic and organic forms have been identified in the Nature. In addition, the selenium can easily form compounds with metals and occurs in about 50 minerals. It is present in four different oxidation states in aqueous and subsurface systems, namely -2, 0, +4 and +6 (K. H. Goh, T. T. Lim, Chemosphere 55, (2004) 849; C. Munier-Lamy, S. Deneux-Mustin, C. Mustin, D. Merlet , J. Berthelin, C. Leyval, J. Environ. Radioactivity (2007) doi:10.1016/j.jenvrad. 2007.04.001 ; M. Simonoff, C. Sergeant, S. poulain, M. S. Pravikoff, Comptes Rendus Chimie (2007) doi :10.1016/J.crci. 2007.02.010). Obviously, the fate and transport of Se in contaminated sites are very much influenced by its chemical form and speciation.

Recently, elemental selenium nanoparticles have been fabricated through various approaches, such as laser ablation, solution-phase approach, vapour-phase growth, electrochemical synthesis, photothermally assisted solution phase, ultrasonic, hydrothermal or solvothermal method and micelle-mediated synthesis J. M. Song, J. H. Zhu, S. H. Yu, J. Phys. Chem. B. 110 (2006) 23790).

Calcium carbonate is an inorganic compound that has been widely studied due to its abundance in nature as a mineral and biomineral. Calcium carbonate particles are found in three polymorph structures, which are generally classified as rhombic calcite, needle-like aragonite and spherical vaterite. Calcite belonging to Trigonal-Hexagonal-Scalenohedral crystallographic class is the most stable phase at room temperature under normal atmospheric conditions, while aragonite and vaterite belong to Orthorombic-Dipyramidal class and Hexagonal-Dihexagonal Dipyramidal class, respectively. The later are metastable polymorphs which readily transform into the stable calcite. The specific formation of one of the polymorphs of crystalline calcium carbonate particles depends mainly on the precipitation conditions, such as pH, temperature and supersaturation. Supersaturation is usually considered to be the main controlling factor (Y. S. Han, G. Hadiko, M. Fuji, M. Takahashi, J. Crystal Growth 276 (2005) 541).

Many experimental studies have been reported about the synthetic precipitation of the various forms of calcium carbonate and the conditions under which these may be produced, including the importance of initial supersaturation, temperature, pressure, pH and hydrodynamics. The effect of impurities and additives has also been well studied (L. Moore, J. D. Hopwood, R. J. Davey, J. Crystal Growth 261 (2004) 93; K. J. Westin, A. C. Rasmuson, J. Colloids Interface Sci. 282 (2005) 370; H. Tsuno, H. Kagi, T. Akagi, Bull. Chem. Soc. Jpn. 74 (2001) 479; Y. Fujita, G. D. Redden, J. Ingram, M. M. Cortez, G. Ferris, R. W. Smith, Geochem. Cosmochem. A. 68 (2004) 3261; S. J. Freij, A. Godelitsas, A. Putnis, J. Crystal Growth 273 (2005) 535; L. A. Gower, D. A. Tirrell, J. Crystal Growth 191 (1998) 153; R. G. Jonasson, K. Rispler, B. Wiwchar, W. D. Gunter, Chem. Geol. 132 (1996) 215; A. Chrissanthopoulos, N. P. Tzanetos, A. K. Andreopoulou, J. Kallitsis, E. Dalas, J. Crystal Growth 280 (2005) 594; M. Menadakis, G. Maroulis, P. G. Koutsoukos, Computational Materials Science 38 (2007) 522; E. Dousi, J. Kallitsis, A. Chrisssanthopoulos, A. H. Mangood, E. Dalas, J. Crystal Growth 253 (2003) 496; L. Pastero, E. Costa, B. Alessandria, M. Rubbo, D. Aquilano, J. Crystal Growth 247 (2003) 472; Y. J. Lee, R. Reeder, Geochem.Cosmochem. A. 70 (2006) 2253; M. Temmam, J. Paquette, H. Vali, Geochem. Cosmochem. A. 64 (2000) 2417; E. Dalas, A. Chalias, D. Gatos, K. Barlos, J. Colloids Interface Sci. 300 (2006) 536; G. Montes-Hernandez, F. Renard, N. Geoffroy, L. Charlet, J. Pironon, J. Crystal Growth 308 (2007) 228).

Searching and designing novel methods to synthesize elemental selenium with controlled morphology is important from the viewpoint of fundamental issues and application. For example, recently, the $Se^0$ nanoparticles are attracting more and more attention due to their excellent high biological activity and lower toxicity in animals and man.

One of the aims of the invention is to provide a process of preparation of an elemental selenium containing composite.

Another aim of the invention is to provide compositions containing elemental selenium.

Another aim is to provide an elemental selenium containing composite allowing the delivery of a bioavailable form of selenium.

Still another aim is to provide a process for synthesizing elemental selenium with controlled morphology.

The present invention relates to the use of a seleno-L-cystine or a derivative thereof in an aqueous suspension of $Ca(OH)_2$ or $Mg(OH)_2$, for preparing an initial dispersion of seleno-L-cystine in said aqueous suspension, said seleno-L-cystine being liable to be fragmented under an appropriate concentration of $O_2$ and to be used for the preparation of a selenium containing composite or compositions containing said composite.

The expression "new selenium containing composites" in this specification means a composite which contains one of the two different forms (red or grey) of elemental selenium (0).

The seleno-Lcystine is constituted of two seleno-L-cysteine molecules bound by a diselenide bridge.

The seleno-L-cysteine is an amino acid present in several enzymes (for example glutathione peroxidases, tetraiodothyronine 5' deiodinases, thioredoxin reductases, formate dehydrogenases, glycine reductases and some hydrogenases).

The terms "seleno-L-cystine", "selenocystine" can be used alternatively and refers to the same meaning.

It can be chemically or biologically synthesized or is commercially available from a lot of suppliers such as Sigma-Aldrich.

By the expression "derivative thereof" is meant any selenium containing compound that can be used instead of seleno-L-cystine.

Examples of selenium derivatives are, but without being limited to them, sodium selenite, seleno methionine, sodium selenate, selenocysteine.

Seleno-L-cystine is the preferred compound but seleno-D-cystine or a mixture of seleno-L-cystine and seleno-D-cystine can also be used.

By "fragmentation" is meant a process of breaking into fragment, for example by hydrolysis of the C-Se bonds.

The term "suspension" means a mixture of finely divided $Ca(OH)_2$ or $Mg(OH)_2$ in water.

According to the invention, $Ca(OH)_2$ or $Mg(OH)_2$ are preferred but the invention is not limited to them. Any other dihydroxide minerals can be used such as $Ba(OH)_2$, $Zn(OH)_2$, $Ba(OH)_2$, provided they are not toxic for human organism.

The term "dispersion" means a mixture in which fine particles of seleno-L-cystine are scattered throughout water.

By « composite » is meant an assembly of at least two non miscible materials giving rise to new properties which are absent from each of the materials when taken alone.

Elemental selenium ($Se°$ or $Se(O)$) can be found under several forms such as a amorphous form the colour of which is red, or a crystalline form, the colour of which is grey.

In a preferred embodiment, the above defined composite is prepared by reaction of said initial dispersion of seleno-L-cystine in an aqueous suspension of $Ca(OH)_2$ or $Mg(OH)_2$ with a gaseous $CO_2$ source.

The carbonation of calcium hydroxide described by the global well-known reaction,

$$Ca(OH)_{2(s)} + CO_{2(aq)} \rightarrow CaCO_{3(s)} + H_2O$$

which is an exothermic process that concerns simultaneously the dissolution of $Ca(OH)_2$,

$$Ca(OH)_{2(s)} \xrightarrow{water} Ca^{2+} + 2OH^-$$

and the dissociation of aqueous $CO_2$,

$$CO_{2(aq)} + H_2O \rightarrow CO_3^{2-} + 2H^+$$

The $CO_2$ source can be pure or concentrated $CO_2$ gas, i.e. $CO_2$ gas containing a small proportion of $SO_x$ or $NO_x$, or a mixture of $CO_2$ gas with an inert gas such as, for example, nitrogen or argon, preferably pure $CO_2$ source, in particular pure $CO_2$ source with a purity of 99.995%.

In a preferred embodiment, the concentration of $O_2$ used in the above defined preparation of said dispersion is comprised from about 1 to about 9 $mg/l_{H2O}$.

9 $mg/l_{H2O}$ is the maximum concentration of oxygen in water in the standard conditions.

Below 1 $mg/l_{H2O}$, the concentration of oxygen is too low to obtain a fragmentation of seleno-L-cystine.

In a more preferred embodiment, the concentration of $O_2$ used in the above defined preparation of said dispersion is comprised from about 1 $mg/l_{H2O}$ to about 7 $mg/l_{H2O}$, preferably from about 5 $mg/l_{H2O}$ to about 7 $mg/l_{H2O}$, in particular 6 $mg/l_{H2O}$.

The $O_2$ concentration used herein corresponds to a low or poor concentration, i.e., a concentration lower than the concentration of $O_2$ in the initial dispersion $Ca(OH)_2$ or $Mg(OH)_2$ and seleno-L-cystine.

In another preferred embodiment, the ratio (w/w) $Ca(OH)_2$ or $Mg(OH)_2$ / seleno-L-cystine used for the above defined preparation of said dispersion is comprised from about 1 to about to 15, in particular 15.

In another preferred embodiment, said selenium containing composite defined above consists of a calcium or magnesium carbonate matrix on which red elemental selenium (Se°) under amorphous form is deposited.

By "calcium or magnesium carbonate matrix" is meant a surrounding substance within which something else originates, develops, or is contained.

The use of $Ca(OH)_2$ leads to a carbonate matrix also named calcite matrix and the use of $Mg(OH)_2$ leads to a magnesium carbonate matrix.

The calcite matrix is constituted by nano- and micro-rhombohedral crystals ($<2\mu m$) and micrometric agglomerates and/or aggregates ($<5\mu m$).

Thus, under a poor concentration of $O_2$ and a ratio $Ca(OH)_2$ or $Mg(OH)_2$ / seleno-L-cystine comprised from about 1 to about to 15, the fragmentation of seleno-L-cystine gives rise to the red amorphous form of elemental selenium which is deposited on the matrix, i.e. present at the surface of the matrix but not included in said matrix.

The terms "adsorbed" or "adsorption" can also be used in this description and have the same meaning that "deposited" or "deposit".

The composite is constituted of spherical selenium nanoparticles ($<500nm$) deposited on the calcite matrix and the spherical Se nanoparticles give a stable red coloration to the composite.

In a more preferred embodiment, the concentration of $O_2$ used in the above defined preparation of said dispersion is comprised from about 7 $mg/l_{H2O}$ to about 9 $mg/l_{H2O}$, in particular 9$mg/l_{H2O}$.

The $O_2$ concentration used herein corresponds to a rich or high concentration, i.e., a concentration corresponding to the initial concentration of $O_2$ in the aqueous $Ca(OH)_2$ or $Mg(OH)_2$ suspension.

According to another preferred embodiment, the ratio (w/w) $Ca(OH)_2$ or $Mg(OH)_2$ / seleno-L-cystine for the above defined preparation of said dispersion is comprised from about 15 to about to 60 , in particular 15.

According to a more preferred embodiment, said composite consists of a calcite matrix containing grey elemental selenium (Se°) under crystalline form.

Thus, under a rich concentration of $O_2$ and a ratio $Ca(OH)_2$ or $Mg(OH)_2$ / seleno-L-cystine comprised from about 15 to about to 60, the fragmentation of seleno-L-cystine gives rise to the grey crystalline form of elemental selenium which is dispersed in the carbonate matrix.

The composite is characterized by hexagonal selenium microparticles ($<25\mu m$) dispersed in the calcite matrix. For this case, a grey coloration of composite is observed.

In another aspect, the present invention relates to a composite of calcite matrix containing elemental selenium under a bioavailable form.

By "bioavailable form" is meant a form of selenium which is absorbed or becomes available at the site of physiological activity after administration.

In a preferred embodiment, the composite above defined is constituted of said elemental selenium under amorphous form deposited on said calcite matrix.

The observed coloration of the composite is therefore red and is stable during several weeks of storage at room conditions and without protection from the light.

In a preferred embodiment, the composite above defined is constituted of said elemental selenium under a crystalline form dispersed in said calcite matrix.

The observed coloration of the composite is therefore grey and is stable during several weeks of storage at room conditions and without protection from the light.

According to another aspect, the present invention relates to a dispersion of seleno-L-cystine containing oxidized selenocystine under $SeO_3^{2-}$ form, in an aqueous suspension $Ca(OH)_2$ or $Mg(OH)_2$.

The seleno-L-cystine in $Ca(OH)_2$ alkaline solution undergoes a partial oxidation, before the introduction of $CO_2$ into said initial dispersion, to Se (IV) and Se(VI) due to the alkaline medium and dissolved oxygen.

In a preferred embodiment, the atomic selenium ratio (w/w) $SeO_3^{2-}$/ selenocystine of the above defined dispersion is comprised from about 0.01 to about 0.25, in particular 0.25, wherein the concentration of $O_2$ is comprised from about 1 $mg/l_{H2O}$ to about 7 $mg/l_{H2O}$, preferably from about 5 $mg/l_{H2O}$ to about 7 $mg/l$ $H_2O$, in particular 6 $mg/l_{H2O}$.

The liquid chromatography coupled to ICPMS detection revealed that selenocystine was rapidly fragmented in $Ca(OH)_2$ alkaline solutions (pH $\cong$ 12.5 measured at 25°C). Here, about 25% of initial atomic selenium contained in the selenocystine (experiment with 200mg/kg$_{water}$) was oxidized to Se(IV) under $O_2$-poor conditions. (Table 1).

**Table 1**.

| t min | SeCyst | SeIV | SeVI | $pH_{25°C}$ |
|---|---|---|---|---|
| 0 | 0.86 ± 0.08 | 24 ± 1 | nd | 12.60 |
| 2 | nd | 9.1 ± 0.7 | nq | 12.43 |
| 6 | 8.9 ± 0.4 | 0.37 ± 0.01 | nq | 7.44 |
| 10 | 9.3 ± 0.1 | 0.95 ± 0.02 | nd | 7.30 |
| 30 | 4.0 ± 0.2 | 1.7 ± 0.2 | nq | 7.34 |
| 60 | nd | 2.5 ± 0.1 | nd | 7.10 |

The HPLC/ICPMS measurements of twelve solution samples of a hydrothermal carbonation-selonocystine fragmentation experiment under $O_2$ poor concentration and 200 mg/kg $_{water}$ of seleno cystine. Concentrations are given in mg (Se) l$^{-1}$

t: reaction time concerning the experiment (t=0 "before $CO_2$ injection in the reactor"); SeCyst: seleno-L-cystine; SeIV: selenite ($SeO_3^{-2}$); SeVI: selenate ($SeO_4^{-2}$); $pH_{25°C}$: pH measured at 25°C; nd: not detected; nq: not quantifiable.

In a more preferred embodiment, the atomic selenium ratio (w/w) $SeO_3^{2-}$/selenocystine of the above defined dispersion is comprised from about 0.26 to about 0.40, in particular 0.40, wherein the concentration of $O_2$ is comprised from about 7 mg/l$_{H2O}$ to about 9 mg/l$_{H2O}$, in particular 9mg/l$_{H2O}$.

Conversely, about 40% of initial atomic selenium contained in the selenocystine (experiment with 100mg/kg$_{water}$) was mainly oxidized to Se(IV) and a slight amount oxidized to (VI) under $O_2$-rich conditions (Table 2).

**Table 2**.

| t min | SeCyst | SeIV | SeVI | $pH_{25°C}$ |
|---|---|---|---|---|
| 0 | nd | 17 ± 1 | 0.21 ± 0.02 | 12.63 |
| 2 | nd | 4.7 ± 0.2 | 0.27 ± 0.01 | 12.58 |
| 6 | 0.68 ± 0.05 | 1.5 ± 0.1 | 0.22 ± 0.2 | 8.03 |
| 10 | 0.65 ± 0.05 | 4.0 ± 0.3 | 0.27 ± 0.02 | 7.30 |
| 30 | 0.15 ± 0.03 | 5.6 ± 0.2 | 0.25 ± 0.02 | 7.28 |
| 60 | nd | 5.7 ± 0.3 | 0.24 ± 0.02 | 7.15 |

The HPLC/ICPMS measurements of twelve solution samples of a hydrothermal carbonation-selonocystine fragmentation experiment under $O_2$ rich concentration and 100 mg/kg $_{water}$ of seleno cystine. Concentrations given in mg (Se) l$^{-1}$

t: reaction time concerning the experiment (t=0 "before $CO_2$ injection in the reactor"); SeCyst: seleno-L-cystine; SeIV: selenite ($SeO_3^{-2}$); SeVI: selenate ($SeO_4^{-2}$); $pH_{25°C}$: pH measured at 25°C; nd: not detected; nq: not quantifiable.

According to another aspect, the present invention relates to a food composition comprising an elemental selenium containing composite wherein the dosage of said composite is appropriate for an intake from 11 μg/day to 400μg/day, preferably from 55 to 70 μg/day.

Selenium is necessary for the human organism but is toxic at high doses.

According to Lucia Letavayová (Toxicology, Volume 227, Issues 1-2, 3 October 2006, Pages 1-14) an intake of <11 μg/day results in deficiency problems and based on human studies, intakes of 400 μg/day were established as the maximum safe dietary dose with no observed adverse effect.

According to J. E. Spallholz et al. (Sci. Total Environ. 323, 21-32 (2004), the human daily dosage for men is 70 μg/day and for women 55 μg/day.

The selenium contained in the composite of the food composition of the invention can be red or grey.

According to another aspect, the present invention relates to a pharmaceutical composition comprising an elemental selenium containing composite wherein the dosage of said composite is appropriate for an administration from 10 μg/day to 70 μg/day, preferably from 55 to 70 μg/day.

The selenium contained in the composite of the pharmaceutical composition can be red or grey.

In another aspect, the present invention relates to a process of preparation of a selenium containing composite comprising a carbonation step of an initial dispersion of seleno-L-cystine in an aqueous suspension of Ca(OH)$_2$ or Mg(OH)$_2$ with an appropriate concentration of $O_2$.

By "carbonation step" is meant a reaction step of seleno-L-cystine in an aqueous suspension of Ca(OH)$_2$ or

$Mg(OH)_2$ with a $CO_2$ source as defined above to obtain a matrix of a mineral carbonate such as a calcite or magnesium carbonate, i.e. an elemental selenium containing composite.

In a preferred embodiment, the above defined process of preparation of the composite, comprises the following steps:

> **a)** introducing seleno-L-cystine in an aqueous suspension of $Ca(OH)_2$ or $Mg(OH)_2$ to obtain a dispersion of seleno-L-cystine in said aqueous suspension of $Ca(OH)_2$ or $Mg(OH)_2$,
> **b)** optionnally introducing an inert gas into said dispersion at a pressure comprised from 50 bars to 90 bars, in particular 90 bars during 15 to 60 minutes, in particular 30 min, to obtain a pressurized dispersion,
> **c)** heating the above dispersion obtained in step a) or b) at a temperature from about 70°C to about 90°C, in particular to about 90°C to obtain a heated optionally pressurized dispersion,
> **d)** in case when an inert gas has been introduced, purging said heated pressurized dispersion until atmospheric pressure to obtain a heated depressurized dispersion in which the $O_2$ concentration is lower than the $O_2$ concentration at atmospheric pressure,
> **e)** introducing into said heated dispersion or heated depressurized dispersion a gaseous $CO_2$ source and reacting during 10 to 30 minutes, in particular 30 minutes to obtain an aqueous suspension of a selenium containing composite,
> **f)** cooling, depressurising and centrifuging the above said aqueous suspension to obtain said selenium containing composite.

The inert gas introduced optionally in step b) can be any inert gas, preferably heavier than $O_2$ such as Ar, Kr et Xe.

Macroscopically, the typical color of calcite is white, while the typical colors of elemental selenium are grey and red.

The introduction of the inert gas followed by heating and then purging allows the partial substitution of $O_2$ by the inert gas and thus lowers the concentration of $O_2$ dissolved in the dispersion.

Therefore, depending on the optional introduction of Ar or not, the elemental selenium obtained is under a crystalline form or an amorphous form respectively and thus the composite is grey or red respectively.

Table 1 shows that before the $CO_2$ injection in the reactor (*t=0*), the selonocystine was slightly detected in the solution, but the kinetic data revealed a selenocystine desorption/liberation process from $Ca(OH)_2$ particles during carbonation process (*t≠0*), this effect being preferentially noticed under $O_2$-poor conditions.

This suggests that a significant proportion of the initial SeCyst was adsorbed onto the $Ca(OH)_2$ particles. Consequently, at higher adsorbed quantity of SeCyst, i.e. under $O_2$-poor conditions, the precipitation/growth of spherical nanoparticles of red elemental selenium was favoured during the carbonation process.

Conversely, at lower adsorbed quantity of SeCyst, i.e. under O-rich conditions, the precipitation/growth of hexagonal microparticles of grey elemental selenium was favored during carbonation process.

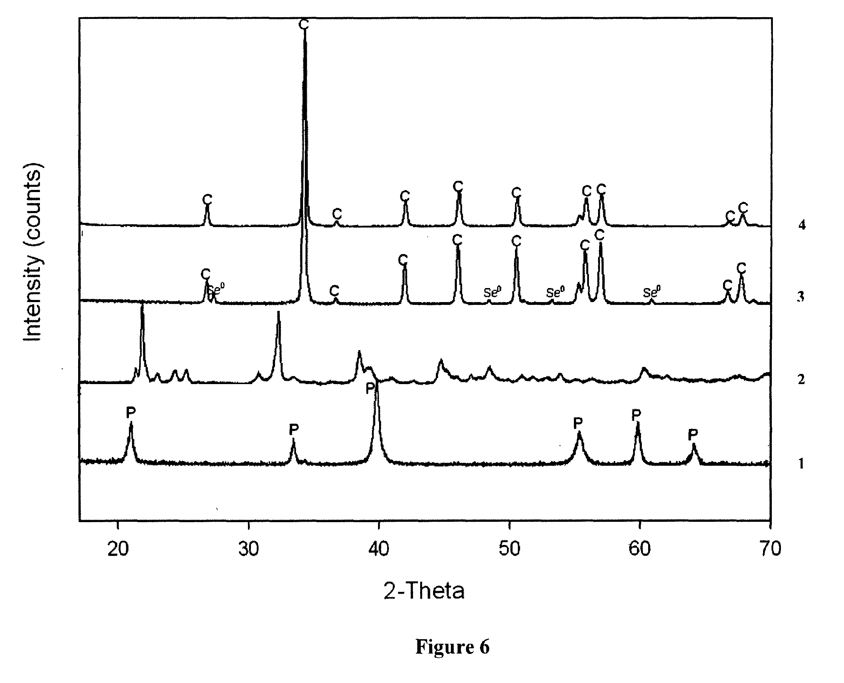

The selenite-oxyanion produced during heating of $Ca(OH)_2$ suspension follows a complex path during carbonation process. Firstly, its solution concentration was drastically decreased by adsorption/incorporation on/in calcite matrix, leading to the unusual morphologies for calcite "ex. star-like morphologies" (Figure 6). And secondly, a slight concentration increase was observed caused by a dissolution process of calcite fine particles due to a high molar excess of $CO_2$ in the system.

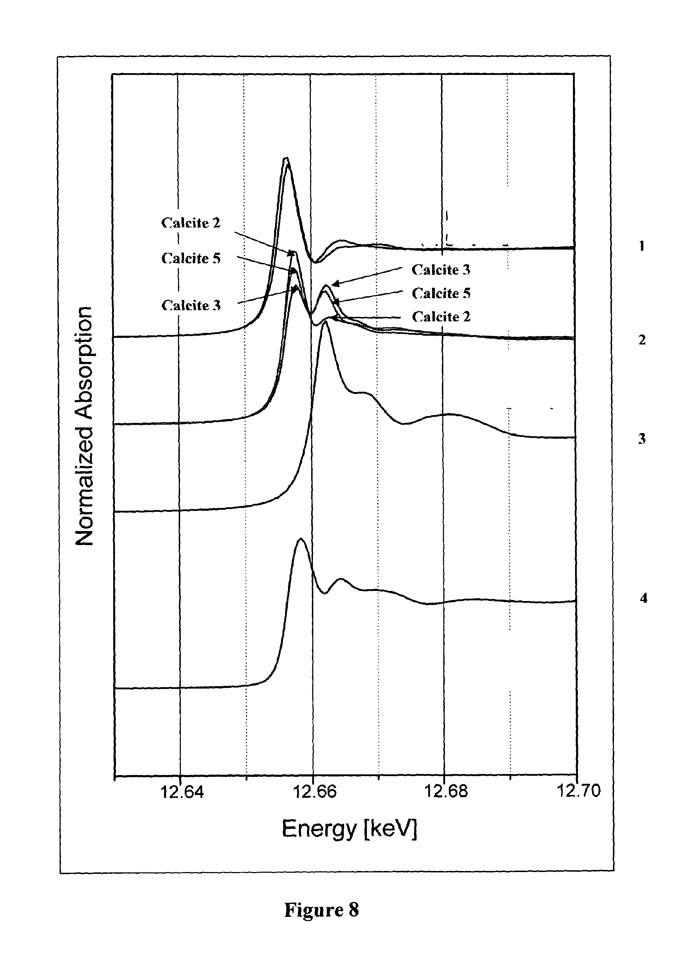

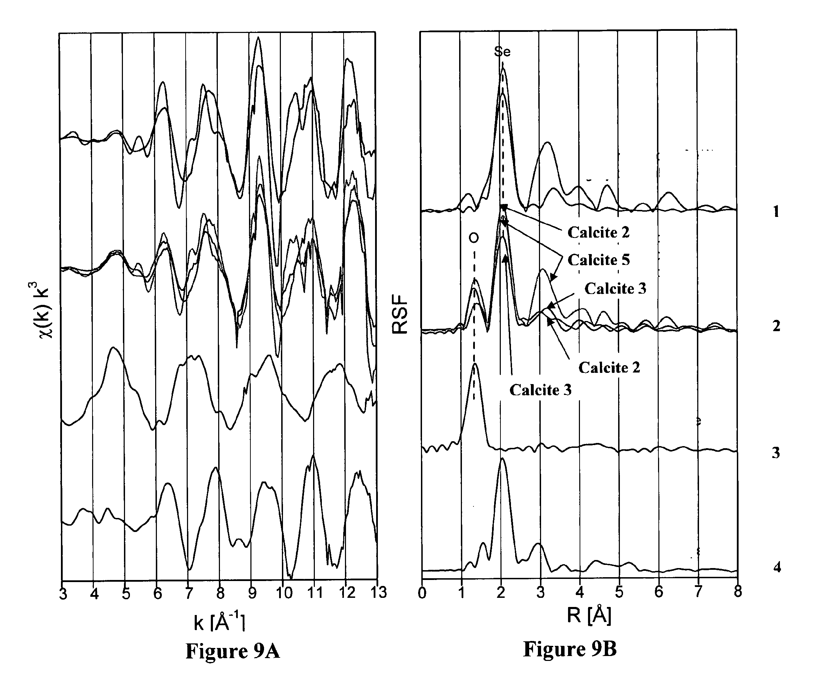

The adsorption and/or incorporation of selenite ($SeO_3^{-2}$) on/in the calcite were supported by XANES and EXAFS spectrometry. This powerful technique allowed also the identification and quantification of different selenium forms co-existing with calcite for three selected solid samples (labelled calcite 2, calcite 3 and calcite 5 in figures 8 and 9 and Table 2).

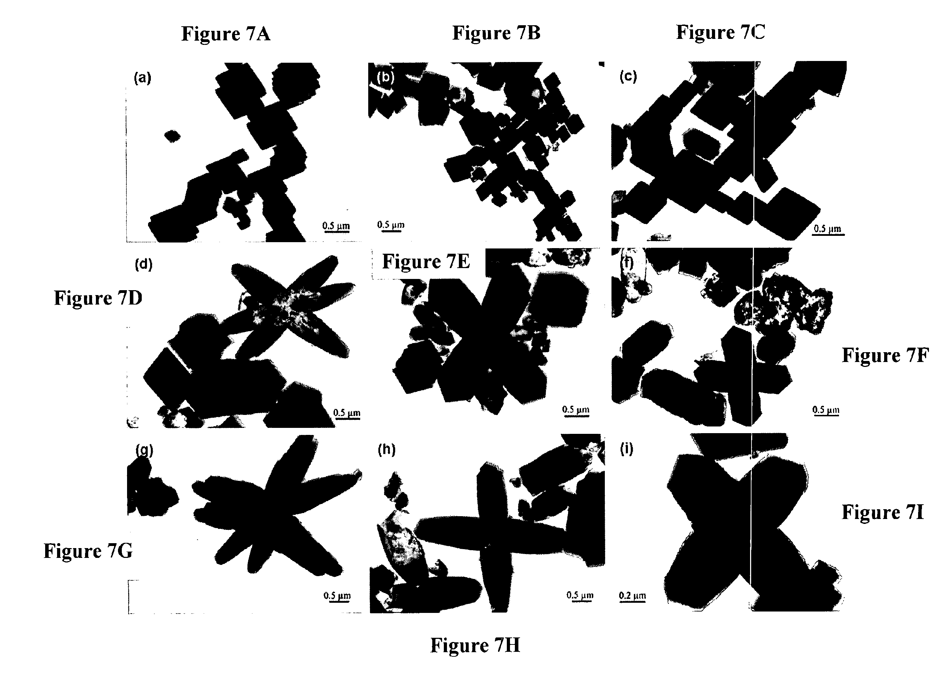

The Se K-edge XANES spectra of the three calcite samples are dominated by a white line at 12,656.5 eV, indicative of elemental Se (Figure 7.) (L. Charlet, A. C. Scheinost, C. Tournassat, J. M. Greneche, A. Gehin, A. Fernandez-Martinez, S. Coudert, D. Tisserand, J. Brendle, Geochim. Cosmochim. Acta 71 (2007) 5731).

Since the white line peak position of selenocystine is about 2 eV above that of elemental Se, it can be discarded that substantial amounts of selenocystine is present in the samples. Samples 3 and 5 have an additional strong oscillation at 12,662 eV, which coincides with the white line of Se(IV), indicating that these samples contain tetravalent Se, in addition to elemental Se.

The corresponding Se K-edge EXAFS are shown in Figure 8. The Fourier transform peaks at about 2.1 Å (uncorrected for phase shift) in the spectra of elemental Se and of selenocystine are due to Se-Se backscattering within the coordination sphere. This is also the strongest peak in the Fourier transform spectra of the three calcite samples.

Red and grey elemental Se are discernable by the intensity of the 1st and 2nd shells, the ones for grey Se being much stronger due to the higher structural order. The spectrum of sample 5 is very similar to that of grey Se, while samples 2 and 3 are closer to red Se.

The Se-O backscattering peak of the Se(IV) reference, $Na_2SeO_3$, occurs at a smaller distance of 1.4 Å. All three sample spectra show a contribution from this shell, although much lower in intensity than the pure Se (IV) compound. It can therefore be concluded that all samples contain a small amount of Se(IV) adsorbed/incorporated onto/into calcite in addition to Se(0). This is in agreement with XANES results.

To further support this tentative phase identification and to derive quantitative information, linear combination fits were performed of the $k^3$-weighted EXAFS spectra (Table 2). In confirmation of the visual interpretation of EXAFS spectra, sample calcite 5 contains a large amount of grey Se, while the other two samples are dominated by red Se.

Note that satisfying fits were achieved only, when a reference spectrum of the aqueous selenite was added for samples calcite 2 and calcite 3, and the reference spectrum of a Se(IV) solid phase was added for sample calcite 5. The relative amount of Se(IV) corresponds to the relative height of the Se-O Fourier peak (Figure 8).

**Table 2 Linear combination fit of $k^3$-weighted EXAFS spectra**.

| Sample | Se grey | Se red | Se(IV) | Sum |
|--------|---------|--------|--------|-----|
| Calcite 2 | 0.02 | 0.87 | 0.12 | 1.01 |
| Calcite 3 | 0.15 | 0.50 | 0.28 | 0.93 |
| Calcite 5 | 0.74 | 0.07 | 0.23 | 1.04 |

Calcite 2: experiment under $O_2$-poor conditions, 100 mg/kg$_{water}$ of selenocystine, 1 hour of reaction.
Calcite 3: experiment under $O_2$-poor conditions, 50 mg/kg$_{water}$ of selenocystine, 1 hour of reaction.
Calcite 5: experiment under $O_2$-rich conditions, 100 mg/kg$_{water}$ of selenocystine, 1 hour of reaction.
Se(IV) correspond to Se(IV)$_{aquo}$ and $Na_2SeO_3$ references in Charlet et al. (L. Charlet, A. C. Scheinost, C. Tournassat, J. M. Greneche, A. Gehin, A. Fernandez-Martinez, S. Coudert, D. Tisserand, J. Brendle, Geochim. Cosmochim. Acta 71 (2007) 5731).

Thereofore, the available quantity of oxygen in the suspension controls the fragmentation process of the selenocystine, leading to the precipitation/growth of elemental selenium with two different morphologies and different sizes of particles.

Consequently, it is demonstrated that the fragmentation process of selenocystine under $O_2$-poor or $O_2$-rich conditions participates to the precipitation/growth of calcite with unusual morphologies.

In a preferred embodiment the concentration of $O_2$ of the above process of preparation is comprised from about 1 mg/l$_{H2O}$ to about 7 mg/l$_{H2O}$, preferably from about 5 mg/l$_{H2O}$ to about 7 mg/l $H_2O$, in particular 6 mg/l$_{H2O}$.

The elemental selenium obtained is under an amorphous form and thus the composite is red.

In a preferred embodiment the concentration of $O_2$ of the above process of preparation is comprised is comprised from about 7 mg/l$_{H2O}$ to about 9 mg/l$_{H2O}$, in particular 9mg/l$_{H2O}$.

The elemental selenium obtained here is under a crystalline form and thus the composite is grey.

In a more preferred embodiment the above defined process of preparation comprises the following steps:

**a)** introducing seleno-L-cystine, in an aqueous suspension of $Ca(OH)_2$ or $Mg(OH)_2$, the ratio (w/w) $Ca(OH)_2$ or $Mg(OH)_2$/ seleno-L-cystine being comprised from about 1 to about to 15, in particular 15, to obtain an initial dispersion of seleno-L-cystine,
**b)** introducing a inert gas such as Ar into said dispersion at a pressure from about 50 to about 90 bars, in particular 90 bars, during from about 15 min to 60 min, in particular 30 min to obtain to obtain a pressurized dispersion,
**c)** heating the above pressurized dispersion at a temperature from about 70°C to about 90°C, in particular 90°C during from about 30 to about 120 min, preferably from about 60 to about 90 min, in particular 60 min, while maintaining the pressure of step b) constant, to obtain a heated pressurized dispersion,
**d)** purging said heated pressurized dispersion until atmospheric pressure to obtain a heated depressurized dispersion in which the $O_2$ concentration is lower than the $O_2$ concentration at atmospheric pressure,
**e)** introducing into said heated depressurized dispersion, a gaseous $CO_2$ source, the ratio (w/w) $Ca(OH)_2$ or $Mg(OH)_2$/ $CO_2$ being comprised from about from about 0.6 to about 0.15, preferably from about 0.3 to about 0.2, in particular 0.21, and adjusting the pressure from about 70 bars to about 90 bars, in particular 90 bars with Ar and reacting during 10 to 30 minutes, in particular 30 minutes to obtain an aqueous suspension of a selenium containing composite,
**f)** cooling at about 35°C, depressurizing and centrifuging said aqueous suspension to obtain said selenium containing composite.

The amorphous form of the elemental selenium depends not only on the concentration of $O_2$ but also on the ratio (w/w) $Ca(OH)_2$ or $Mg(OH)_2$/ seleno-L-cystine which must be comprised between 1 and 15.

At this ratio, the pressure of the Ar introduced allows to reduce the $O_2$ concentration. The Ar pressure must be comprised from 50 bars to 90 bars, preferably from 60 to 90 bars, more preferably from 70 to 90 and preferably from 80 to 90 bars, in particular 90 bars.

Nevertheless, the pressure must be higher than 50 bars in order to obtain red selenium. If the pressure is lower than 50 bars, the concentration of $O_2$ is still too high to obtain red selenium and the elemental selenium obtained is grey.

The total reaction time comprises introducing Ar into said dispersion and heating said dispersion, purging pressurized heated dispersion, introducing $CO_2$ and reacting, cooling at about 35°C, depressurizing and centrifuging to obtain said composite.

The total reaction time is therefore comprised from about 100 min to about 255 min, preferably from about 150 to about 220, in particular 200 min.

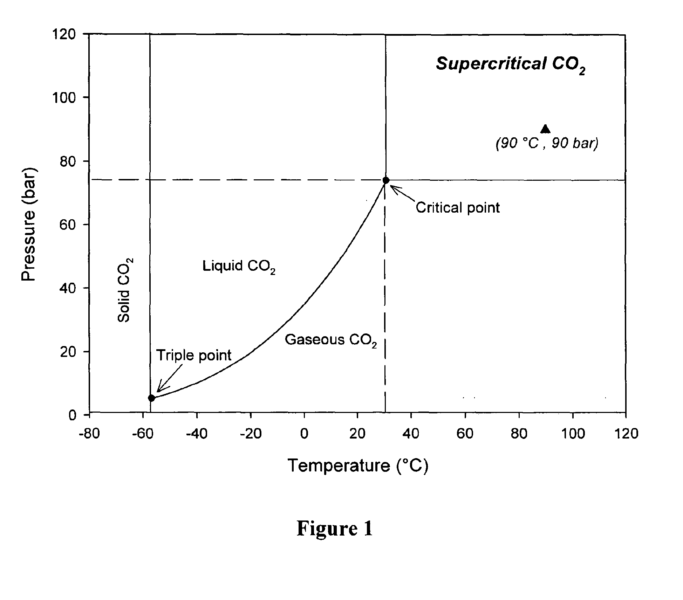

The $CO_2$ source is introduced in excess and allows acidifying the pressurized heated dispersion. If the ratio is above 0.6, said dispersion will be too acid and the reaction will not run. Under these temperature and pressure conditions, the vapour phase consists mainly of an Ar and $CO_2$ mixture with the $CO_2$ in a supercritical state (see Figure 1). The pressure and temperature control is critical for the control of the size and the structure of the particles of the matrix.

The red coloration of composite starts to be observable when 50 $mg/kg_{water}$ are used and the colour intensity increases with an increase of selenocystine dose. This coloration behaviour was reproducible at a constant calcium hydroxide dose (3 $g/kg_{water}$ for this study). The coloration of composite was stable in the aqueous suspension even after several weeks of storage at room conditions and without protection from the light.

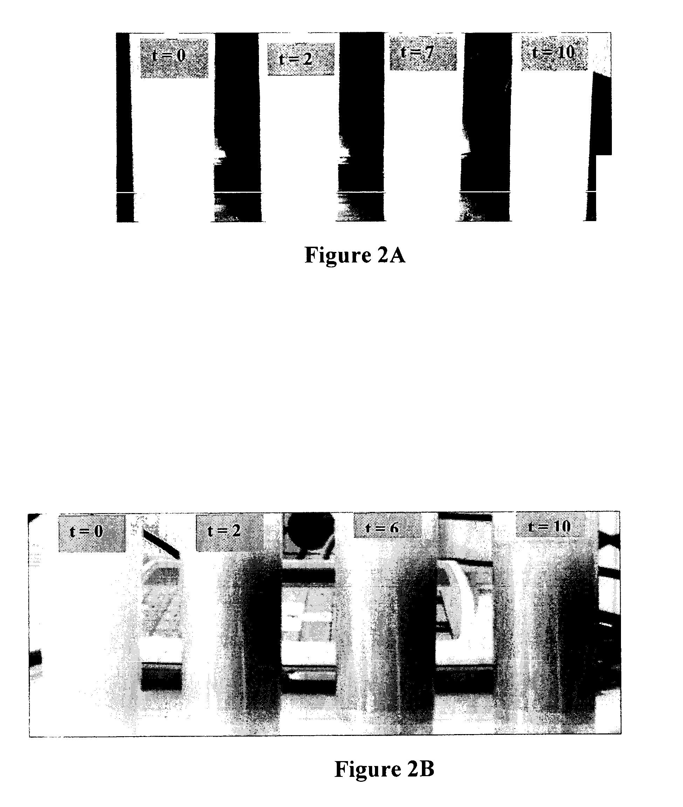

Microscopically, the red composite was mainly characterized by spherical selenium nanoparticles (< 500 nm) deposited on the calcite matrix. The carbonate matrix was constituted by nano- and micro rhombohedral crystals (< 2 $\mu$m) and micrometric agglomerates and/or aggregates (< 5 $\mu$m) (Figure 2).

In a more preferred embodiment the above defined process of preparation comprises the following steps:

**a)** introducing seleno-L-cystine, in an aqueous suspension of $Ca(OH)_2$ or $Mg(OH)_2$, the ratio (w/w) $Ca(OH)_2$ or $Mg(OH)_2$/ seleno-L-cystine being comprised from about 15 to about to 60, in particular 15, to obtain an initial dispersion of seleno-L-cystine,

**b)** heating the above dispersion at about 70°C to about 90°C, in particular 90°C during from about 30 min to about 120 min, preferably from about 60 min to about 90 min, in particular 60 min, while maintaining the pressure of step b) constant, to obtain a heated dispersion,

**c)** introducing into said heated dispersion, a gaseous $CO_2$ source, the ratio (w/w) $Ca(OH)_2$ or $Mg(OH)_2$/ $CO_2$ being comprised from about from about 0.6 to about 0.15, preferably from about 0.3 to about 0.2, in particular 0.21, and adjusting the pressure from about 70 bars to about 90 bars, in particular 90 bars with Ar and reacting during 10 to 30 minutes, in particular 30 minutes to obtain an aqueous suspension of a selenium containing composite,

**d)** cooling at about 35°C, depressurizing and centrifuging said aqueous suspension to obtain said selenium containing composite.

The crystalline form of the elemental selenium depends not only on the concentration of $O_2$ but also on the ratio (w/w) $Ca(OH)_2$ or $Mg(OH)_2$/ seleno-L-cystine which must be higher than 15.

In this case, the optional step of introducing Ar must not be done and the dispersion cited above is directly heated to 90°C at atmospheric pressure.

The following steps are identical as above.

The total reaction time comprises heating said dispersion, introducing $CO_2$ and reacting, cooling at about 35°C, depressurizing and centrifuging to obtain said composite.

The total reaction time is therefore comprised from about 85 min to about 195 min, preferably from about 100 to about 160, in particular 135 min.

The grey intensity is slightly dependent on the selenocystine dose from 50 to 200 $mg/kg_{water}$. The composite coloration was also stable in the aqueous suspension even after several weeks of storage at room conditions and without protection from the light.

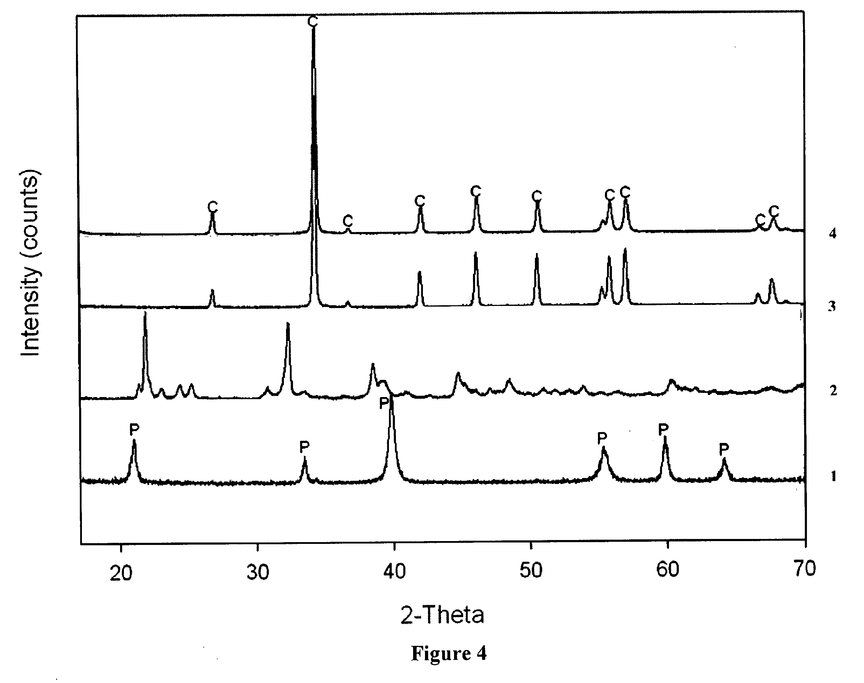

Microscopic observations showed that the composite was characterized by elongated hexagonal selenium microparticles (<25$\mu$m) dispersed in the calcite matrix (Figure 4).

## DESCRIPTION OF THE FIGURES

**Figure 1** shows the experimental Pressure-Temperature conditions represented on a pressure-temperature phase

diagram for $CO_2$.

**Figures 2A and 2B** represent the coloration change of elemental selenium containing composite at different selenocystine doses at different times of reaction with $CO_2$ and under $O_2$-poor conditions. It is to be noted that the elemental selenium containing composite was manually dispersed to take these photographs.

Figure 2A: composite obtained with 50 mg/kg of selenocystine (grey elemental selenium containing composite).
From left to right tubes : t = 0, t = 2, t = 7, t = 10 min
Figure 2B: 200 mg/kg of selenocystine (red elemental selenium containing composite obtained).
From left to right tubes : t = 0, t = 2, t = 6, t = 10 min.

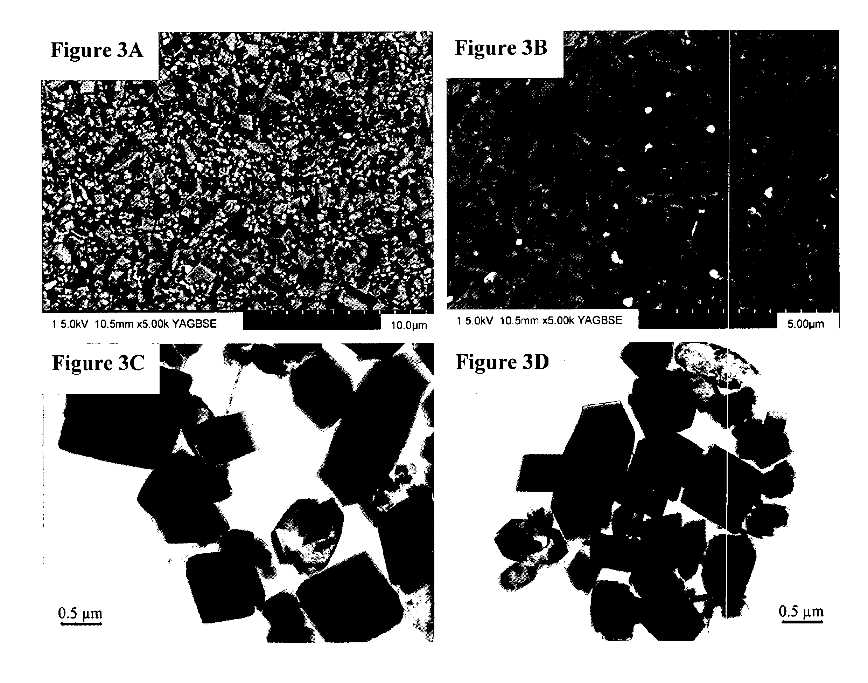

**Figures 3A, 3B, 3C and 3D** represent the red elemental selenium containing composite synthesized by hydrothermal carbonation of calcium hydroxide coupled with a complex selenocystine fragmentation under $O_2$-poor conditions.
Figures 3A and 3B: Backscattering SEM observations "without metal coating";
Figures 3C and 3D: TEM micrographs.

**Figure 4** represents XRD measurements of starting solid materials (calcium hydroxide and selenocystine) and solid products (pure calcite and red elemental selenium containing composite). Hydrothermal carbonation of calcium hydroxide under $O_2$-poor conditions (P=90 bars, T=90 °C; $Ca(OH)_2$ dose=3 $g/kg_{water}$; Selenocystine dose=200 $mg/kg_{water}$; 1 hour of reaction time with $CO_2$. P: Portlandite, C: Calcite).
The lower graph **1** represents the starting Ca $(OH)_2$: portlandite.

The graph **2** represents the starting selenocystine.
The graph **3** represents the red elemental selenium composite synthesized with 200 mg of selenocystine.
The upper graph **4** represents the calcite synthesized without 200 mg of selenocystine.

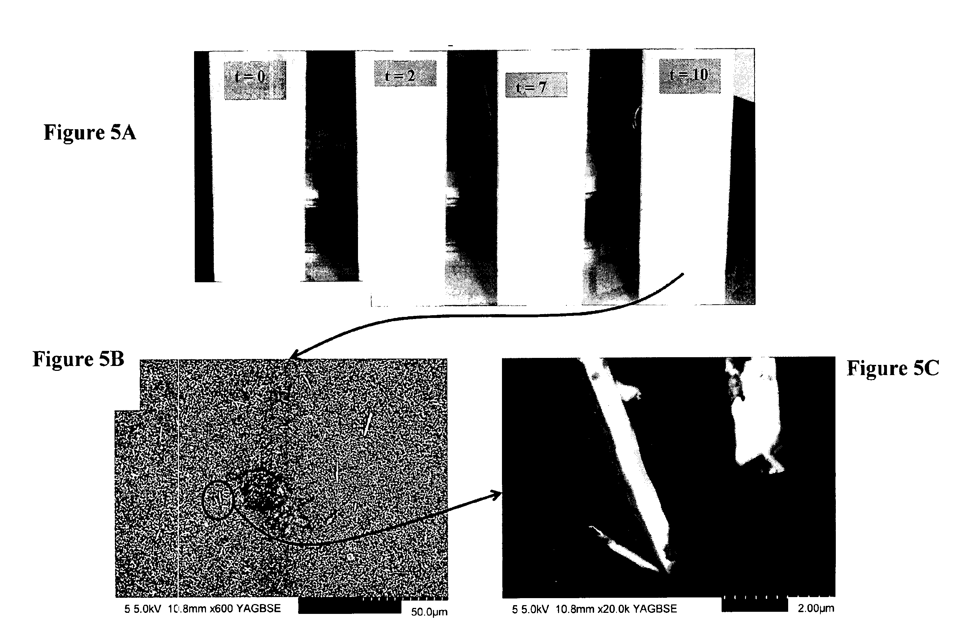

**Figures 5A, 5B, 5C** represent the coloration change of grey elemental selenium containing composite synthesized by hydrothermal carbonation with $CO_2$ of calcium hydroxide with selenocystine, at different times of reaction and under $O_2$-rich conditions
Figure 5A: composite obtained with 200 mg/kg selenocystine (grey elemental selenium containing composite).
From left to right tubes : t = 0, t = 2, t = 7, t = 10 min.
Figure 5B and 5C: Backscattering SEM observations "without metal coating" of the composite obtained with tube t = 10 min,

**Figure 6 represents** XRD measurements of starting solid materials (calcium hydroxide and selenocystine) and solid products (pure calcite and gray $Se^0$/calcite composite). Hydrothermal carbonation of calcium hydroxide under $O_2$-rich conditions (P=90 bars, T=90 °C; $Ca(OH)_2$ dose=3 $g/kg_{water}$; selenocystine dose=200 $mg/kg_{water}$; 1 hour of reaction time with $CO_2$. P: portlandite, C: calcite.
The lower graph **1** represents the starting Ca $(OH)_2$: portlandite.

The graph **2** represents the starting selenocystine.
The graph **3** represents the red elemental selenium composite synthesized with 200 mg of selenocystine.
The upper graph **4** represents the calcite synthesized without 200 mg of selenocystine.

**Figures 7A, 7B, 7C, 7D, 7E, 7F, 7G, 7H and 7I** represent the hydrothermal carbonation process without or with initial selenocystine dose (200 $mg/kg_{water}$)
Figures 7A, 7B and 7C: rhombohedral nano- and micro-particles of calcite (hydrothermal carbonation process without initial selenocystine dose).
Figures 7D, 7E, 7F, 7G, 7H and 7I: unusual morphology for calcite "star-like morphologies" (with initial selenocystine dose 200 $mg/kg_{water}$).

**Figure 8** represents the Se K-edge XANES of three calcite samples in comparison to select references.

The graphs **1** have been obtained with the reference of Se(0) red amorphous and Se(0) grey trigonal.
The graphs **2** have been obtained with calcite 2, 3 and 5:

Calcite 2: experiment under $O_2$-poor conditions, 100 $mg/kg_{water}$ of selenocytine, 1 hour of reaction;
Calcite 3: experiment under $O_2$-poor conditions, 50 $mg/kg_{water}$ of selenocytine, 1 hour of reaction.
Calcite 5: experiment under $O_2$-rich conditions, 100 $mg/kg_{water}$ of selenocytine, 1 hour of reaction.

The graph **3** has been obtained with $Na_2SeO_3$.
The graph **4** has been obtained with the selenocystine reference.

**Figure 9A and 9B** represent the Se K-edge EXAFS spectra (Fig 9A: left) and corresponding Fourier transforms (Fig 9B: right) of the three calcite samples and select references.

The graphs **1** have been obtained with the reference of Se(0) red amorphous (lower graph and Se(0) grey trigonal (upper graph).

The graphs **2** have been obtained with calcite 2, 3 and calcite 5:

Calcite 2, experiment under $O_2$-poor conditions, 100 mg/kg$_{water}$ of selenocytine, 1 hour of reaction;
Calcite 3, experiment under $O_2$-poor conditions, 50 mg/kg$_{water}$ of selenocytine, 1 hour of reaction.
Calcite 5, experiment under $O_2$-rich conditions, 100 mg/kg$_{water}$ of selenocytine, 1 hour of reaction.

The graph **3** has been obtained with $Na_2SeO_3$.
The graph **4** has been obtained with the selenocystine reference.

## EXAMPLES

### Example 1: Precipitation/growth of calcite with unusual morphologies

For these experiments, the hydrothermal carbonation of calcium hydroxide described by the global well-known reaction,

$$Ca(OH)_{2(s)} + CO_{2(aq)} \rightarrow CaCO_{3(s)} + H_2O$$

is an exothermic process that concerns simultaneously the dissolution of $Ca(OH)_2$,

$$Ca(OH)_{2(s)} \xrightarrow{water} Ca^{2+} + 2OH^-$$

and the dissociation of aqueous $CO_2$,

$$CO_{2(aq)} + H_2O \rightarrow CO_3^{2-} + 2H^+$$

These processes produce a fast supersaturation ($S_l$) of the solution with respect to calcite,

$$S_l = \frac{(Ca^{2+})(CO_3^{2-})}{K_{sp}} > 1$$

where ($Ca^{2+}$) and ($CO_3^{2-}$) are the activities of calcium and carbonate ions in the solution, respectively, and $K_{sp}$ is the thermodynamic solubility product of calcite. Then, the nucleation stage (formation of nuclei or critical cluster) takes place in the system,

$$Ca^{2+} + CO_3^{2-} \rightarrow CaCO_3 \ (nuclei)$$

The sampling with time of the suspensions in the reactor allowed the identification of the nucleation stage, characterized by the formation of an apparent stable emulsion after about two minutes of reaction time.
Finally, the crystal growth occurred spontaneously until the equilibrium calcite and the solution was reached,

$$CaCO_3 \ (nuclei) \rightarrow CaCO_3 \ (calcite)$$

For this study, it was demonstrated that the selenocystine fragmentation during heating and carbonation process had insignificant effect on the $Ca(OH)_2$-calcite conversion.

### Example 2 : Synthesis of elemental selenium composite Under $O_2$-poor conditions or system with a purge step.

One liter of high-purity water with electrical resistivity of 18.2 MΩ·cm, 3 g of commercial portlandite $Ca(OH)_2$

(calcium hydroxide provided by Sigma-Aldrich) with 96% chemical purity (3% $CaCO_3$ and 1% other impurities) and different quantities (0, 50, 100 or 200 mg) of seleno-L-cystine $CO_2HCH(NH_2)CH_2(Se)_2CH_2CH(NH_2)CO_2H$ (provided by Sigma-Aldrich) with chemical purity (≥98.0%) were placed in a titanium reactor (Parr© autoclave with internal volume of two liters). The hydroxide and selenocystine particles were immediately dispersed with mechanical agitation (400 rpm) to obtain a dispersion of seleno-L-cystine in an aqueous suspension of calcium hydroxide.

Then, at room temperature the gas argon with 99.999% chemical purity (provided by Linde Gas S.A.) was injected into the reaction cell in order to control the pressure at 90 bars during 30 minutes.

After this time period, the suspension was heated to 90°C with a furnace adapted to the reactor. During heating stage the pressure increased into the system, but it was kept constant at about 90 bars by using successive manual purge until the temperature was stabilized (about 90 minutes).

Then, about 20 ml of suspension were sampled in the reactor ($t=0$) and immediately a flash purge was carried out until atmospheric pressure was reached. The argon adsorption in the suspension, the heating stage and the gas purge, allowed a partial removal of dissolved oxygen from the suspension ($O_2$-poor conditions).

When the atmospheric pressure was reached in the reactor, 14.5 g of $CO_2$ with 99.995% chemical purity (provided by Linde Gas S.A.) were injected in the reactor and the total pressure in the system was immediately adjusted to 90 bar by argon injection and reacting during 30 minutes.

After this, a semi-batch system (sampling with time) was performed in order to measure the pH (using MA235 pH/ion analyzer) and, calcium and selenium concentration (using ICP Perkin Elmer Optima 3300 DV) in filtered solutions. For this case, about 20 ml of suspension were sampled in the reactor as a function of time (t=2, 6, 10, 30 and 60 minutes) during composite formation. It is to be noted that the pH measurement was carried out at 25 °C after filtration, cooling and degasification of the solutions

At the end of the experiment, the autoclave was removed from the heating system and immersed in cold water. The reaction cell was depressurized during the water cooling period. After water cooling at 35°C (about 15 minutes) the autoclave was disassembled, and the solid product was carefully recovered and separated by centrifugation (30 minutes at 12,000 rpm), decanting the supernatant solutions.

Finally, the solid product was dried directly in the centrifugation flasks for 72 h at 65°C, manually recovered and stocked in plastic flasks.

The carbonation yield based on the $Ca(OH)_2$-$CaCO_3$ transformation is about 96 % estimated by mass balance. XRD measures show a complete conversion of $Ca(OH)_2$ and a complete selenocystine fragmentation because, after carbonation, no more $Ca(OH)_2$ is observed and no traces of selenocystine have been found.

**Example 3: Characterization of selenium containing composite produced under $O_2$-poor conditions**

The microstructure characterization of the red composite by using x-ray diffraction, suggests a complete $Ca(OH)_2$-calcite conversion, i.e. that the metastable crystalline phases of $CaCO_3$, such as vaterite and aragonite were not produced during the $Ca(OH)_2$ carbonation process in the experiments of the invention. Concerning the selenocystine, this crystalline organic material was not identified on the x-ray diffraction spectra for red selenium containing composite (Figure 3). This suggests a complete chemical transformation or water dissolution of the selenocystine in this experiment and explains the precipitation of red nano-particles of elemental selenium (observed by SEM and TEM microscopy).

**Example 4: Synthesis of elemental selenium composite under $O_2$-rich conditions or system without a purge step.**

The dispersion of seleno-L-cystine in an aqueous suspension of calcium hydroxide of example 2 was directly heated to 90°C at atmospheric pressure. During heating stage the pressure increased into the system, but it was kept constant at about 90 bars by using successive manual purge until the temperature was stabilized (about 90 minutes). When the temperature was stabilized, about 20 ml of suspension were sampled in the reactor ($t=0$).

Then, 14.5 g of $CO_2$ were injected into the reactor and the total pressure in the system was immediately adjusted at 90 bars by argon injection and reacting during 30 minutes.

After the pressure setting, the sampling and composite recovery procedures were identical to the $O_2$-poor system described above (exemple 1).

The carbonation yield based on the $Ca(OH)_2$-$CaCO_3$ transformation is about 96 % estimated by mass balance. XRD measures show a complete conversion of $Ca(OH)_2$ and a complete selenocystine fragmentation because, after carbonation, no longer $Ca(OH)_2$ is observed and no traces of selenocystine have been found.

**Example 5: Characterization of selenium containing composite produced under $O_2$-rich conditions**

For these experiments, it was demonstrated that a grey composite can be produced under $O_2$-rich conditions.

For this case, the grey intensity seems to be slightly dependent on the selenocystine dose from 50 to 200 $mg/kg_{water}$. The composite coloration was also stable in the aqueous suspension even after several weeks of storage at room conditions and without protection from the light.

Microscopic observations showed that the composite was characterized by elongated hexagonal selenium microparticles (<25μm) dispersed in the calcite matrix (Figure 4). In addition, the X-ray diffraction measurements support these microscopic observations. It is well known that the trigonal or hexagonal morphologies are the most stable crystalline phases for $Se^0$. Here, a small proportion contained in a given composite can be identified by X-ray diffraction measurements (Figure 5).

This figure also shows a complete $Ca(OH)_2$-Calcite conversion under $O_2$-rich conditions, revealing that the oxygenated conditions in the suspension have not significant effect on the carbonation process efficiency.

**Claims**

1. Use of a seleno-L-cystine or a derivative thereof in an aqueous suspension of $Ca(OH)_2$ or $Mg(OH)_2$, for preparing an initial dispersion of seleno-L-cystine in said aqueous suspension, said seleno-L-cystine being liable to be fragmented under an appropriate concentration of $O_2$ and to be used for the preparation of a selenium containing composite or compositions containing said composite.

2. Use according to claim 1, wherein said composite is prepared by reaction of said initial dispersion with a gaseous $CO_2$ source.

3. Use according to claim 1 or 2, wherein the concentration of $O_2$ is comprised from about 1 to about 9 $mg/l_{H2O}$.

4. Use according to anyone of claims 1 to 3, wherein the concentration of $O_2$ is comprised from about 1 $mg/l_{H2O}$ to about 7 $mg/l_{H2O}$, preferably from about 5 $mg/l_{H2O}$ to about 7 mg/l $H_2O$, in particular 6 $mg/l_{H2O}$.

5. Use according to claim 4, wherein the ratio (w/w) $Ca(OH)_2$ or $Mg(OH)_2$ / seleno-L-cystine is comprised from about 1 to about to 15, in particular 15.

6. Use according to claim 5, wherein said selenium containing composite consists of a calcium or magnesium carbonate matrix on which red elemental selenium (Se°) under amorphous form is deposited.

7. Use according to anyone of claims 1 to 3, wherein the concentration of $O_2$ is comprised from about 7 $mg/l_{H2O}$ to about 9 $mg/l_{H2O}$, in particular 9$mg/l_{H2O}$.

8. Use according to claim 7, wherein the ratio (w/w) $Ca(OH)_2$ or $Mg(OH)_2$ / seleno-L-cystine is comprised from about 15 to about to 60 , in particular 15.

9. Use according to claim 8, wherein said composite consists of a calcite matrix containing a grey elemental selenium (Se°) under crystalline form.

10. Composite of calcite matrix containing elemental selenium under a bioavailable form.

11. Composite according to claim 10, wherein said elemental selenium is under amorphous form and is deposited on said calcite matrix.

12. Composite according to claim 10, wherein said elemental selenium is under a crystalline form and is dispersed in said calcite matrix.

13. Dispersion of seleno-L-cystine containing oxidized selenocystine under $SeO_3^{2-}$ form, in an aqueous suspension Ca $(OH)_2$ or $Mg(OH)_2$.

14. Dispersion according to claim 13, wherein the atomic selenium ratio (w/w) $SeO_3^{2-}$/selenocystine is comprised from about 0.01 to about 0.25, in particular 0.25, wherein the concentration of $O_2$ is comprised from about 1 $mg/l_{H2O}$ to about 7 $mg/l_{H2O}$, preferably from about 5 $mg/l_{H2O}$ to about 7 mg/l $H_2O$, in particular 6 $mg/l_{H2O}$.

15. Dispersion according to claim 13, wherein the atomic selenium ratio (w/w) $SeO_3^{2-}$/selenocystine is comprised from about 0.26 to about 0.40, in particular 0.40, wherein the concentration of $O_2$ is comprised from about 7 mg/$l_{H2O}$ to about 9 mg/$l_{H2O}$, in particular 9mg/$l_{H2O}$.

16. Food composition comprising an elemental selenium containing composite wherein the dosage of said composite is appropriate for an intake from 11 $\mu$g/day to 400$\mu$g/day, preferably from 55 to 70 $\mu$g/day.

17. Pharmaceutical composition comprising an elemental selenium containing composite wherein the dosage of said composite is appropriate for an administration from 11 $\mu$g/day to 400 $\mu$g/day, preferably from 55 to 70 $\mu$g/day.

18. Process of preparation of a selenium containing composite comprising a carbonation step of an initial dispersion of seleno-L-cystine in an aqueous suspension of $Ca(OH)_2$ or $Mg(OH)_2$ with an appropriate concentration of $O_2$.

19. Process of preparation according to claim 18, comprising the following steps:

   a) introducing seleno-L-cystine in an aqueous suspension of $Ca(OH)_2$ or $Mg(OH)_2$ to obtain a dispersion of seleno-L-cystine in said aqueous suspension of $Ca(OH)_2$ or $Mg(OH)_2$,
   b) optionnally introducing an inert gas into said dispersion at a pressure comprised from 50 bars to 90 bars, in particular 90 bars during 15 to 60 minutes, in particular 30 min, to obtain a pressurized dispersion,
   c) heating the above dispersion obtained in step a) or b) at a temperature from about 70°C to about 90°C, in particular to about 90°C to obtain a heated optionally pressurized dispersion,
   d) in case when an inert gas has been introduced, purging said heated pressurized dispersion until atmospheric pressure to obtain a heated depressurized dispersion in which the $O_2$ concentration is lower than the $O_2$ concentration at atmospheric pressure,
   e) introducing into said heated dispersion or heated depressurized dispersion a gaseous $CO_2$ source and reacting during 10 to 30 minutes, in particular 30 minutes to obtain an aqueous suspension of a selenium containing composite,
   f) cooling, depressurising and centrifuging the above said aqueous suspension to obtain said selenium containing composite.

20. Process of preparation according to claim 18 or 19, wherein the concentration of $O_2$ is comprised from about 1 mg/$l_{H2O}$ to about 7 mg/$l_{H2O}$, preferably from about 5 mg/$l_{H2O}$ to about 7 mg/l $H_2O$, in particular 6 mg/$l_{H2O}$.

21. Process of preparation according to claim 18 or 19, wherein the concentration of $O_2$ is comprised from about 7 mg/$l_{H2O}$ to about 9 mg/$l_{H2O}$, in particular 9mg/$l_{H2O}$.

22. Process of preparation according to anyone of claims 18 to 20, comprising the following steps:

   a) introducing seleno-L-cystine, in an aqueous suspension of $Ca(OH)_2$ or $Mg(OH)_2$, the ratio (w/w) $Ca(OH)_2$ or $Mg(OH)_2$/ seleno-L-cystine being comprised from about 1 to about to 15, in particular 15, to obtain an initial dispersion of seleno-L-cystine,
   b) introducing a inert gas such as Ar into said dispersion at a pressure from about 50 to about 90 bars, in particular 90 bars, during from about 15 min to 60 min, in particular 30 min to obtain to obtain a pressurized dispersion,
   c) heating the above pressurized dispersion at a temperature from about 70°C to about 90°C, in particular 90°C during from about 30 to about 120 min, preferably from about 60 to about 90 min, in particular 60 min, while maintaining the pressure of step b) constant, to obtain a heated pressurized dispersion,
   d) purging said heated pressurized dispersion until atmospheric pressure to obtain a heated depressurized dispersion in which the $O_2$ concentration is lower than the $O_2$ concentration at atmospheric pressure,
   e) introducing into said heated depressurized dispersion, a gaseous $CO_2$ source, the ratio (w/w) $Ca(OH)_2$ or $Mg(OH)_2$/ $CO_2$ being comprised from about from about 0.6 to about 0.15, preferably from about 0.3 to about 0.2, in particular 0.21, and adjusting the pressure from about 70 bars to about 90 bars, in particular 90 bars with Ar and reacting during 10 to 30 minutes, in particular 30 minutes to obtain an aqueous suspension of a selenium containing composite,
   f) cooling at about 35°C, depressurizing and centrifuging said aqueous suspension to obtain said selenium containing composite.

23. Process of preparation according to anyone of claims 18, 19 or 21, under a rich $O_2$ atmosphere, comprising the following steps:

**a)** introducing seleno-L-cystine, in an aqueous suspension of $Ca(OH)_2$ or $Mg(OH)_2$, the ratio (w/w) $Ca(OH)_2$ or $Mg(OH)_2$/ seleno-L-cystine being comprised from about 15 to about to 60, in particular 15, to obtain an initial dispersion of seleno-L-cystine,

**b)** heating the above dispersion at about 70°C to about 90°C, in particular 90°C during from about 30 min to about 120 min, preferably from about 60 min to about 90 min, in particular 60 min, while maintaining the pressure of step b) constant, to obtain a heated dispersion,

**c)** introducing into said heated dispersion, a gaseous $CO_2$ source, the ratio (w/w) $Ca(OH)_2$ or $Mg(OH)_2$/ $CO_2$ being comprised from about from about 0.6 to about 0.15, preferably from about 0.3 to about 0.2, in particular 0.21, and adjusting the pressure from about 70 bars to about 90 bars, in particular 90 bars with Ar and reacting during 10 to 30 minutes, in particular 30 minutes to obtain an aqueous suspension of a selenium containing composite,

**d)** cooling at about 35°C, depressurizing and centrifuging said aqueous suspension to obtain said selenium containing composite.

**Figure 1**

**Figure 2A**

**Figure 2B**

Figure 3B

1 5.0kV 10.5mm x5.00k YAGBSE

5.00μm

Figure 3A

1 5.0kV 10.5mm x5.00k YAGBSE

10.0μm

Figure 3D

0.5 μm

Figure 3C

0.5 μm

2-Theta

Figure 4

Intensity (counts)

Figure 5A

Figure 5B

5 5.0kV 10.8mm x600 YAGBSE          50.0μm

5 5.0kV 10.8mm x20.0k YAGBSE          2.00μm

Figure 5C

**Figure 6**

Figure 7A, Figure 7B, Figure 7C, Figure 7D, Figure 7E, Figure 7F, Figure 7G, Figure 7H, Figure 7I

**Figure 8**

Figure 9A

Figure 9B

**European Patent Office**

**EUROPEAN SEARCH REPORT**

Application Number

EP 08 29 0234

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DE 201 16 346 U1 (ORTHOMOL PHARMAZEUTISCHE VERTR [DE]) 20 December 2001 (2001-12-20) * example 4 * | 16 | INV. A23L1/304 A61K33/04 C01B19/00 C01B19/02 C01F11/18 |
| X | US 6 090 414 A (PASSWATER RICHARD A [US] ET AL) 18 July 2000 (2000-07-18) * column 9, line 22 - line 55; claims 2-7 * | 16 | |
| X | GAO X ET AL: "HOLLOW SPHERE SELENIUM NANOPARTICLES: THEIR IN-VITRO ANTI HYDROXYL RADICAL EFFECT" ADVANCED MATERIALS, WILEY VCH, WEINHEIM, DE, vol. 14, no. 4, 19 February 2002 (2002-02-19), pages 290-293, XP001132509 ISSN: 0935-9648 * the whole document * | 17 | |
| X | EP 1 762 233 A (SCHIELEIN FELIX [DE]) 14 March 2007 (2007-03-14) * example 1 * | 17 | TECHNICAL FIELDS SEARCHED (IPC) A23L A61K C01B C01F |
| A | MONTES-HERNANDEZ ET AL: "Calcite precipitation from CO2-H2O-Ca(OH)2 slurry under high pressure of CO2" JOURNAL OF CRYSTAL GROWTH, ELSEVIER, AMSTERDAM, NL, vol. 308, no. 1, 2 October 2007 (2007-10-02), pages 228-236, XP022283212 ISSN: 0022-0248 Section 2.1 Carbonation of calcium hydroxide (stirred reactor), Section 3 Results and discussion | 1-15, 18-23 | |

-/--

The present search report has been drawn up for all claims

| Place of search | Date of completion of the search | Examiner |
|---|---|---|
| Munich | 19 August 2008 | Schmitt, Ruth |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

European Patent
Office

**EUROPEAN SEARCH REPORT**

Application Number

EP 08 29 0234

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | MONTES-HERNANDEZ G ET AL: "Textural properties of synthetic nano-calcite produced by hydrothermal carbonation of calcium hydroxide" JOURNAL OF CRYSTAL GROWTH, ELSEVIER, AMSTERDAM, NL, vol. 310, no. 11, 19 February 2008 (2008-02-19), pages 2946-2953, XP022640578 ISSN: 0022-0248 * page 2947 - page 2951 *<br>----- | 1-15, 18-23 | |
| A | J.-S. ZHANG, X.-Y. GAO, L.-D. ZHANG, Y.-P. BAO: "Biological effects of a nano red elemental selenium" BIOFACTORS, vol. 15, 2001, pages 27-38, XP009104615 * the whole document *<br>----- | 1-15, 18-23 | |
| A | Q. LU, F. GAO, S. KOMARNENI: "Cellulose-directed Growth of Selenium Nanobelts in Solution" CHEM. MATER., vol. 18, 2006, pages 159-163, XP002492556 2. Experimental Section Synthetic Processes<br>----- | 1-15, 18-23 | TECHNICAL FIELDS SEARCHED (IPC) |
| T | G. MONTES-HERNANDEZ, A. FERNANDEZ-MARTINEZ, L. CHARLET, F. RENARD, A.C. SCHEINOST, M. BUENO: "Synthesis of a SeO/Calcite Composite Using hydrothermal Carbonation of Ca(OH)2 Coupled to a Complex Selenocystine Fragmentation" CRYSTAL GROWTH AND DESIGN, vol. 8, no. 7, 28 May 2008 (2008-05-28), pages 2497-2504, XP002492557 * the whole document *<br>----- | 1-23 | |

The present search report has been drawn up for all claims

| Place of search | Date of completion of the search | Examiner |
|---|---|---|
| Munich | 19 August 2008 | Schmitt, Ruth |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons
..................................................................
& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 08 29 0234

This annex lists the patent family members relating to the patent documents cited in the above-mentioned European search report.
The members are as contained in the European Patent Office EDP file on
The European Patent Office is in no way liable for these particulars which are merely given for the purpose of information.

19-08-2008

| Patent document cited in search report | | Publication date | Patent family member(s) | | Publication date |
|---|---|---|---|---|---|
| DE 20116346 | U1 | 20-12-2001 | NONE | | |
| US 6090414 | A | 18-07-2000 | NONE | | |
| EP 1762233 | A | 14-03-2007 | AT | 390131 T | 15-04-2008 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **J. M. Song ; J. H. Zhu ; S. H. Yu.** *J. Phys. Chem. B.,* 2006, vol. 110, 23790
- **Y. Ding ; Q. Li ; Y. Jia ; L. Chen ; J. Xing ; Y. Qian.** *J. Crystal Growth,* 2002, vol. 241, 489
- **Y. Bai ; Y. Wang ; Y. Zhou ; W. Li ; W. Zheng.** *Materials Letters,* 2007
- **E. Kapolna ; M. Shah ; J. A. Caruso ; P. Fodor.** *Food Chemistry,* 2007, vol. 101, 1398
- **A. Tarze ; M. Dauplais ; I. Grigoras ; M. Lazard ; N. T. Ha-Duong ; F. Barbier ; S. Blanquet ; P. Plateau.** *J. Biol. Chem.,* 2007, vol. 282 (12), 8759
- **R. Caputo ; S. Capone ; M. Della Greca ; L. Longobardo ; G. Pinto.** *Tetrahedron Letters,* 2007, vol. 48, 1425
- **D. Peng ; J. Zhang ; Q. Liu ; E. Will Taylor.** *J. Inorg. Biochem.,* 2007, vol. 101, 1457
- **J. E. Spallholz ; D. J. Hoffman.** *Aquatic Toxicology,* 2002, vol. 57, 27
- **M. S. Stewart ; J. E. Spallholz ; K. H. Neldner ; B. C. Pence.** *Free Radic. Biol. Med,* 1999, vol. 26, 42
- **M. Ochsenkuhn-Petropoulou ; F. Tsopelas.** *Anal. Chim. Acta,* 2002, vol. 467, 167
- **K. H. Goh ; T. T. Lim.** *Chemosphere,* 2004, vol. 55, 849
- **C. Munier-Lamy ; S. Deneux-Mustin ; C. Mustin ; D. Merlet ; J. Berthelin ; C. Leyval.** *J. Environ. Radioactivity,* 2007, doi:10.1016/j.jenvrad.2007.04.001
- **M. Simonoff ; C. Sergeant ; S. poulain ; M. S. Pravikoff.** *Comptes Rendus Chimie,* 2007, doi :10.1016/J.crci.2007.02.010
- **Y. S. Han ; G. Hadiko ; M. Fuji ; M. Takahashi.** *J. Crystal Growth,* 2005, vol. 276, 541
- **L. Moore ; J. D. Hopwood ; R. J. Davey.** *J. Crystal Growth,* 2004, vol. 261, 93
- **K. J. Westin ; A. C. Rasmuson.** *J. Colloids Interface Sci.,* 2005, vol. 282, 370
- **H. Tsuno ; H. Kagi ; T. Akagi.** *Bull. Chem. Soc. Jpn.,* 2001, vol. 74, 479
- **Y. Fujita ; G. D. Redden ; J. Ingram ; M. M. Cortez ; G. Ferris ; R. W. Smith.** *Geochem. Cosmochem. A.,* 2004, vol. 68, 3261
- **S. J. Freij ; A. Godelitsas ; A. Putnis.** *J. Crystal Growth,* 2005, vol. 273, 535
- **L. A. Gower ; D. A. Tirrell.** *J. Crystal Growth,* 1998, vol. 191, 153
- **R. G. Jonasson ; K. Rispler ; B. Wiwchar ; W. D. Gunter.** *Chem. Geol.,* 1996, vol. 132, 215
- **A. Chrissanthopoulos ; N. P. Tzanetos ; A. K. Andreopoulou ; J. Kallitsis ; E. Dalas.** *J. Crystal Growth,* 2005, vol. 280, 594
- **M. Menadakis ; G. Maroulis ; P. G. Koutsoukos.** *Computational Materials Science,* 2007, vol. 38, 522
- **E. Dousi ; J. Kallitsis ; A. Chrisssanthopoulos ; A. H. Mangood ; E. Dalas.** *J. Crystal Growth,* 2003, vol. 253, 496
- **L. Pastero ; E. Costa ; B. Alessandria ; M. Rubbo ; D. Aquilano.** *J. Crystal Growth,* 2003, vol. 247, 472
- **Y. J. Lee ; R. Reeder.** *Geochem.Cosmochem. A.,* 2006, vol. 70, 2253
- **M. Temmam ; J. Paquette ; H. Vali.** *Geochem. Cosmochem. A.,* 2000, vol. 64, 2417
- **E. Dalas ; A. Chalias ; D. Gatos ; K. Barlos.** *J. Colloids Interface Sci.,* 2006, vol. 300, 536
- **G. Montes-Hernandez ; F. Renard ; N. Geoffroy ; L. Charlet ; J. Pironon.** *J. Crystal Growth,* 2007, vol. 308, 228
- **Lucia Letavayová.** *Toxicology,* 03 October 2006, vol. 227 (1-2), 1-14
- **J. E. Spallholz et al.** *Sci. Total Environ.,* 2004, vol. 323, 21-32
- **L. Charlet ; A. C. Scheinost ; C. Tournassat ; J. M. Greneche ; A. Gehin ; A. Fernandez-Martinez ; S. Coudert ; D. Tisserand ; J. Brendle.** *Geochim. Cosmochim. Acta,* 2007, vol. 71, 5731